# EUROPEAN PATENT APPLICATION

(11) **EP 4 821 439 A1**
(43) Date of publication of application: **14.10.2026**
(21) Application number: 25170042.3
(22) Date of filing: 11.04.2025
(51) Int. Cl.: A61B 5/369, A61B 5/00

(54) **METHOD AND DEVICE FOR REMOVAL OF ARTIFACTS FROM MAGNETO-/ELECTROENCEPHALOGRAPHY MULTI-CHANNEL SIGNALS**

(71) Applicant: Université de Genève, 1211 Genève 4 (CH)
(72) Inventor: ROS, Tomás, 1208 Geneva (CH)
(74) Representative: Sammer, Thomas

(57) **Abstract**

The present invention concerns a signal processing method for removal of artifacts from electroencephalography (EEG) and/or magnetoencephalography (MEG) signals by use of Generalized Eigenvalue Decomposition (GEVD), the method comprising the steps of
- obtaining (1) a EEG/MEG multi-channel signal,
- providing (2) a matrix factorization of said multi-channel signal by applying a generalized Eigenvalue decomposition to the signal by
° determining (2.1) a reference covariance matrix (refCOV) to be used as an input value to the Eigenvalue decomposition of said multi-channel signal,
° extracting (2.3) a data covariance matrix (dataCOV) from the multi-channel signal and
° producing (2.4) a generalized Eigenvalue decomposition of said data covariance matrix (dataCOV) based on said reference covariance matrix (refCOV),
- producing (3), by use of said matrix factorization of the multi-channel signal, an output signal corresponding to said multi-channel signal with artifacts in the signal being removed and non-artifact parts of the signal being retained, the step of

- determining the reference covariance matrix (refCOV) being performed by estimating a theoretical reference covariance matrix (refCOV) based on principles of EEG/MEG signal generation, utilizing electrode locations and leadfield theory.

The present invention is also related to a computer readable medium storing a computer program which causes a computer to implement this method as well as to a device for acquiring and/or for processing a EEG/MEG multi-channel signal, the device having means adapted to perform this method.

## Description

### Field of the invention

The present invention pertains to a signal processing method for removal of artifacts from electroencephalography (EEG) and/or magnetoencephalography (MEG) signals by use of Generalized Eigenvalue Decomposition (GEVD). It also pertains to a computer readable medium storing a corresponding computer program as well as to a device for acquiring and/or for processing such a multi-channel signal.

### Background of the invention and state of the art

In general, the present invention is situated in the field of signal processing, in particular in the context of treating signals collected by electroencephalography (EEG) and magnetoencephalography (MEG).

Electroencephalography (EEG) and magnetoencephalography (MEG) are used to monitor electrical cerebral activity for many years already. Despite being a well researched and inexpensive method for cerebral activity monitoring, electroencephalography struggles to expand outside the controlled environments provided by research and clinical settings. One of the main hurdles in extending EEG's use is the presence of signal contaminations of non-cerebral nature, the removal of which often requires the supervision of a trained experimenter, leading to reduced scalability and reliability of EEG-based products and applications. The presence of artifacts can be problematic, simply for usability of EEG data in clinical practice as well as for more advanced applications like the development of brain computer interfaces [1] such as neurofeedback [2], and removal of such of artifacts may be qualified as one of the most important developments needed in EEG research.

In the past, several strategies have been developed to clean the EEG signal. The most popular of prior art strategies for removal of artifacts from EEG signals is independent component analysis (ICA), which is applied on multi-channel EEG data to perform blind source separation and remove independent components from the signal. While effective to remove ocular artifacts [3] and muscular components [4], ICA is computationally demanding, requires a case-by-case visual inspection of components to reject or complex machine learning classifiers [5], and cannot identify Gaussian distributed noise [6]. ICA is therefore not an ideal choice for inexperienced users, large datasets or online analysis.

Another strategy, called artifact subspace reconstruction (ASR), uses a different approach by relying on principal component analysis (PCA) to exclude EEG components exceeding a threshold, defined by taking clean EEG data as a reference [7]. While being faster, relying on a variance sensitive threshold is not per se sufficient to distinguish between signal and noise [8]. Furthermore, since ASR is essentially a PCA, it can only separate components that are orthogonal, which is not a given in artifactual sources.

To overcome the limitations of prior art techniques for removal of artifacts from EEG signals like ICA and ASR, Generalized Eigenvalue Decomposition (GEVD) offers a promising alternative. GEVD is a known matrix factorization technique allowing to decompose the covariance matrix of the EEG data not based on its intrinsic variance, but on the variance of its difference with a reference covariance matrix (refCOV) given as input [9]. As such, during performing the GEVD, the type of identifiable artifacts aren't constrained neither by orthogonality nor by non-gaussianity [6].

Several implementations of methods using GEVD have been proposed in the past for removal of artifacts from EEG signals, ranging from blind source separation [6], identification and removal of ocular artifacts [10], removal of transcranial current stimulation artifacts [11], removal of transcutaneous auricular vagus nerve stimulation artifacts [12, 13] and generic data cleaning [14]. GEVD has also been used for the identification of pathological signals such as epileptic seizures [15] and is a central algorithm in many brain-computer interfaces applications such as common spatial patterns identification [16, 17, 18].

A first central concern in the use of GEVD for removal of artifacts from EEG signals is the way in which the reference covariance matrix (refCOV) is defined prior to the decomposition. In most cases, researchers have to rely on the manual selection of EEG portions considered clean, artifactual, or a signal of specific interest. This "empirical" selection of reference data can be problematic as it relies on user input and therefore has a limited reproducibility. Techniques for the automatic selection of clean data portions have been tested in ASR [19] and could be implemented in GEVD. However, this approach is predicated on the assumption that a clean portion of empirical data exists in the EEG recording, which may not be the case during low-quality recordings, e. g. using "dry" EEG electrodes with high contact impedance, or in environments with persistent electromagnetic noise, e. g. poorly shielded rooms, during electrostimulation or MRI scanning.

A second concern is the determination of the threshold at which an identified component is to be considered artifactual. Approaches for automatic determination of said threshold have been investigated for various de-artifacting techniques [20] and have been implemented in GEVD through the use of multi-channel Wiener filtering [14].

Therefore, the above mentioned solutions according to prior art for removal of artifacts from EEG signals still present several inconveniences. In particular, whilst prior art techniques like ICA and ASR either are computationally demanding, require a case-by-case visual inspection of components to reject, respectively complex machine learning classifiers, and cannot identify Gaussian distributed noise, or rely on a variance sensitive threshold which is not sufficient to distinguish between signal and noise, even the most promising prior art methods for removal of artifacts from EEG signals using GEVD still suffer problems both with determining the reference covariance matrix (refCOV) required prior to decomposition of the signal and with determining the threshold at which an identified component of the signal is to be considered artifactual.

### Objectives of the invention

It is the object of the present invention to overcome the above mentioned difficulties and to realize a signal processing method for removal of artifacts from EEG/MEG signals using GEVD which allows for an improved determination of the reference covariance matrix (refCOV) required prior to decomposition of the signal. Simultaneously, determination of the threshold at which an identified component of the signal is to be considered artifactual shall be improved, too. Furthermore, a method for removal of artifacts from EEG/MEG signals according to the present invention should allow for faster removal of artifacts from EEG/MEG signals as compared to prior art methods and should allow to reduce or even to avoid user input in order to increase reproducibility, i. e. such method should allow for improvements in terms of both speed and accuracy. Moreover, a method for removal of artifacts from EEG/MEG signals according to the present invention should allow for reduction of cost related to such removal of artifacts from EEG/MEG signals.

### Summary of the solution according to the invention

To this effect, the present invention proposes a signal processing method for removal of artifacts from EEG/MEG multi-channel signals, which is characterized by the features enumerated in claim 1 and which allows to achieve the objectives identified above.

In particular, the method according to the present invention is characterized by the fact that determining the reference covariance matrix (refCOV) is performed, without use of empirical data of the EEG/MEG multi-channel signal, by estimating a theoretical spatial reference covariance matrix (refCOV) based on principles of EEG/MEG signal generation, utilizing electrode locations and leadfield theory.

In preferred embodiments of the method, estimating the theoretical spatial reference covariance matrix (refCOV) is done either by use of a given number of standard electrode locations and of a pre-computed leadfield model corresponding to these standard electrode locations or alternatively by determining the locations of a given number of electrodes effectively used for the specific multi-channel signal to be processed and thereafter computing, by use of a leadfield model specifically computed for these electrode locations, a theoretical reference covariance matrix (refCOV) specifically adapted for this multi-channel signal to be processed.

In particularly preferred embodiments of the method, extracting the data covariance matrix (dataCOV) from the acquired empirical multi-channel signal is performed by dividing the acquired multi-channel signal to be processed, by use of adequate time intervals, into time intervals and, if desired/required, recording the divided multi-channel signal, whilst producing the generalized Eigenvalue decomposition of said data covariance matrix (dataCOV) is performed for each data covariance matrix (dataCOV) in each time interval based, across all time intervals, on said reference covariance matrix (refCOV).

In other particularly preferred embodiments of the method, producing the output signal is performed by automatically setting a threshold for Eigenvalue cutoff by iteratively comparing the effect of different threshold values on similarity of the produced EEG/MEG output signal with the theoretical reference covariance matrix (refCOV). Said iterative comparison of the effect of different threshold values in order to automatically set the threshold for Eigenvalue cutoff may be performed on the parts of the signal being retained and/or on the parts of the signal being removed. Moreover, producing the output signal may optionally be performed by using frequency-specific thresholds for Eigenvalue cutoff, each frequency-specific threshold being determined specifically for given frequency band of the acquired multi-channel signal.

Therefore, a method for removal of artifacts from EEG/MEG signals according to the present invention allows to realize an automatic GEVD algorithm for EEG/MEG data cleaning that is capable of both (1) automatic generation of the reference covariance matrix (refCOV) and (2) automatic thresholding.

The present invention also proposes a computer readable medium storing a computer program which causes a computer to implement the method according to present invention when the program is executed by the computer.

Furthermore, the present invention also proposes a device for acquiring a multi-channel signal by electro-encephalography (EEG) and/or magnetoencephalography (MEG) and/or for processing a multi-channel signal acquired by electroencephalography (EEG) and/or magnetoencephalography (MEG), the device comprising at least an input/output unit, a storage unit allowing to store acquired multi-channel signals, a processing unit allowing to process acquired multi-channel signals, wherein the device is equipped with means adapted to perform the method for removal of artifacts from EEG/MEG multi-channel signals according to the present invention.

Other features and advantages of the present invention are mentioned in the dependent claims as well as in the description disclosing in the following, with reference to the figures, the invention in more detail.

### Brief description of the figures

The attached figures exemplarily and schematically illustrate the principles as well as several embodiments of the present invention.
Figure 1 schematically illustrates the main steps of a method for removal of artifacts from EEG/MEG signals according to the present invention.
Figures 2a and 2b schematically illustrate a comparison between an empirical spatial covariance matrix estimated from clean EEG/MEG resting-state data (figure 2a) and a theoretical spatial covariance matrix computed from the leadfield matrix of a Boundary Element Method (BEM) EEG/MEG forward model (figure 2b), both matrices being normalised channel x channel, i. e. 0 mean with a standard deviation of 1.
Figure 3 schematically illustrates the optional steps for automatically setting a threshold for Eigenvalue cutoff by iteratively comparing the effect of different threshold values on similarity of the produced EEG/MEG output signal with the theoretical reference covariance matrix (refCOV).
Figure 4 schematically illustrates the principle of linearly decomposing an EEG/MEG multi-channel signal.
Figure 5 shows the results of a comparison between a method according to the present invention and three prior art methods in terms of reconstruction accuracy.
Figures 6a to 6d show the results of this comparison by indicating the aggregate means and 95% confidence intervals across all artifact types for SNR, in figure 6a, for RRMSE, in figure 6b, for correlation, in figure 6c, and for average processing time, in figure 6d.
Figure 7 is a table summarizing the results of this comparison in terms of specific characteristics of the investigated denoising techniques.

### Detailed description of the invention

In the following, the invention shall be described in detail with reference to the above mentioned figures.

As already mentioned before, the present invention relates to the field of signal processing, namely to treating signals collected by electroencephalography (EEG) and/or magnetoencephalography (MEG). For the sake of simplifying the language and of increasing readability of the following description, terms like "EEG signals", "EEG data", "EEG/MEG recording", "EEG/MEG signals" or the like are in the following used, except if explicitly indicated differently, such as to generically refer to signals acquired both by electroencephalography (EEG) and/or by magnetoencephalography (MEG). Furthermore, the terms "removal of artifacts", "denoising" and "cleaning", or the like, are used synonymously.

### Basic principle

In general, a method for removal of artifacts from EEG/MEG signals using GEVD according to the present invention proposes to use a so-called Generalized Eigenvalue De-Artifacting Instrument (GEDAI) algorithm which distinguishes from previous methods using GEVD by determining the reference covariance matrix (refCOV), required prior to the decomposition, from principles of EEG/MEG signal generation, utilizing electrode locations and leadfield theory. Specifically, instead of selecting portions of empirical EEG/MEG data like in prior art methods, whether it be clean or artifactual data, to estimate the reference covariance matrix (refCOV), said GEDAI algorithm focuses on identifying theoretically what the covariance of electrodes should be in clean EEG/MEG data. The estimation of the refCOV matrix in the GEDAI algorithm is performed by using the electrodes location to estimate the theoretical spatial covariance matrix from the EEG/MEG leadfield matrix. This theoretical approach to construction of the reference covariance matrix (refCOV) is based on the linear relation between the leadfield dipole covariance and the covariance estimated from electrodes-based time series generated from those dipoles. Given that the reference covariance matrix (refCOV) is theoretical, it is independent of the actual noise-distribution present within an EEG/MEG recording that requires denoising, i. e. it is noise-agnostic. Consequently, the EEG/MEG recording may be 100% temporally contaminated, i. e. with artifacts during every time sample, as long as there are still some cerebral components linearly present within the EEG/MEG signal.

Moreover, in a preferred embodiment of a method for removal of artifacts from EEG/MEG signals using GEVD according to the present invention, said GEDAI algorithm automatically sets the threshold for Eigenvalues cutoff without use of "ground truth" data. Instead, it compares how different thresholding strengths affect the similarity of the cleaned EEG/MEG data covariance with the theoretical template formed by the reference covariance matrix (refCOV). Specifically, this comparison estimates a so-called Signal & Noise Subspace Alignment Index (SENSAI) across different Eigenvalue thresholds, which is done by computing the subspace similarities between the top Eigenvectors of the cleaned and removed signal, respectively, and the top Eigenvectors of the reference matrix (refCOV), i. e. the cosine of the angle between these vectors. With this approach, a signal that is cleaned at an insufficient threshold strength would have a lower similarity to the theoretical reference template than with a more aggressive threshold. Similarly, a signal cleaned by using an excessively aggressive threshold would have a reduced similarity with the theoretical reference template, as non-artifactual data gets removed. This method can be applied also on the part of the data that gets removed by the cleaning procedure: a potential signal that gets thrown away by an aggressive threshold would increase its resemblance with the reference subspace. Taken together, by maximising the subspace similarity of the signal kept, and/or minimizing the subspace similarity of the part of the signal that is removed, by said GEDAI algorithm at different thresholding strengths, it is possible to identify the optimal thresholding strength.

As an important aspect, it should be noted that automatically setting the threshold for Eigenvalues cutoff as proposed by the present invention is independent of using a theoretically determined reference covariance matrix (refCOV) and may also be used with a reference covariance matrix such as determined by prior art methods. Therefore, the two main features of a preferred embodiment of a signal processing method for removal of artifacts according to the present invention, i. e. theoretically detemining a reference covariance matrix (refCOV) and automatically setting the threshold for Eigenvalues cutoff, are independent of each other and thus may be used separately as well as independently in corresponding methods using only one of these features.

### Structure and functioning

A signal processing method for removal of artifacts from electroencephalography (EEG) and/or magnetoencephalography (MEG) signals by use of Generalized Eigenvalue Decomposition (GEVD) according to the present invention comprises a number of steps known in prior art, namely
- obtaining (1) a multi-channel signal acquired by electroencephalography (EEG) and/or magnetoencephalography (MEG),
- providing (2) a matrix factorization of said multi-channel signal by applying a generalized Eigenvalue decomposition to the signal, providing said matrix factorization of the multi-channel signal comprising the substeps of
   ° determining (2.1) a reference covariance matrix (refCOV) to be used as an input value to the Eigenvalue decomposition of said multi-channel signal,
   ° extracting (2.3) a data covariance matrix (dataCOV) from the acquired empirical multi-channel signal and
   ° producing (2.4) a generalized Eigenvalue decomposition of said data covariance matrix (dataCOV) corresponding to said multi-channel signal based on said reference covariance matrix (refCOV), as well as
- producing (3), by use of said matrix factorization of the multi-channel signal, an output signal corresponding to said multi-channel signal with artifacts in the signal being removed by applying an Eigenvalue cutoff and non-artifact parts of the signal being retained.

Furthermore, the method for removal of artifacts from EEG/MEG signals using GEVD according to the present invention distinguishes from prior art methods due to the fact that it comprises use of the above mentioned Generalized Eigenvalue De-Artifacting Instrument (GEDAI) algorithm. In brief, the GEDAI algorithm allows that the above mentioned step of determining the reference covariance matrix (refCOV) is performed, without use of empirical data of the EEG/MEG multi-channel signal, by estimating a theoretical spatial reference covariance matrix (refCOV) based on principles of EEG/MEG signal generation, utilizing electrode locations and leadfield theory.

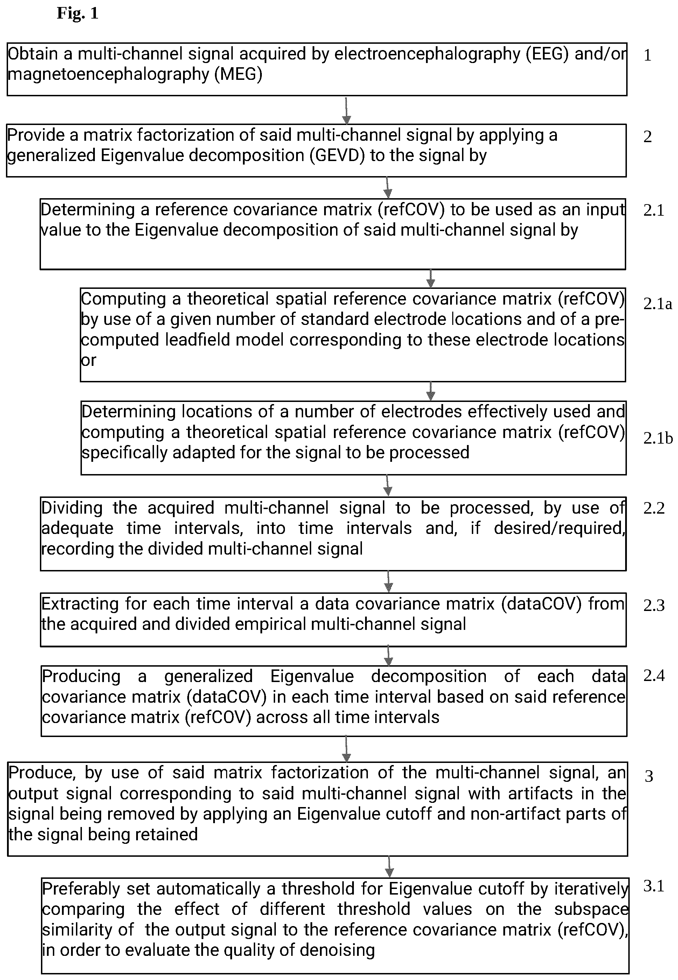

The GEDAI algorithm may be implemented, for example, as a Matlab EEGlab plugin and, in its preferred embodiment, is composed of several steps, summarized in this paragraph as well as schematically shown in figure 1 and explained in detail below. First, a theoretical noise free reference covariance matrix (refCOV) is produced either through a pre-computed leadfield model, which may use for example up to 343 electrodes (known to a person skilled in the art within the framework of a 10-5 system), or through electrode warping onto the patient's scalp surface. Second, the empirical EEG signal (plus presumed noise) to be cleaned is divided into time slices and (if desired or required) recorded, by use of adequate time intervals/slices (usually between 0.5 - 2 seconds in length), and an individual data covariance matrix (dataCOV) is extracted from the signal in each time interval. Each data covariance matrix is then decomposed through a Generalized Eigenvalue Decomposition (GEVD), using said reference covariance matrix (refCOV) as a fixed reference matrix across all time intervals. Then, a so-called SENSAI function (Signal & Noise Subspace Alignment Index) evaluates the output EEG/MEG signal at different magnitudes of Eigenvalue cutoffs to evaluate the quality of denoising. The cutoff is applied to all Eigenvalues (and their associated Eigenvectors) of the interval-by-interval covariance matrices as a threshold for component rejection, as zeroing an Eigenvalue leads to the removal of the component it represents when reconstructing the signal.

### 1. Estimation of the leadfield-based noise-free reference matrix

The proposed GEDAI algorithm offers two options for estimation of the theoretical reference covariance matrix (refCOV). The first option is to pre-compute a theoretical reference covariance matrix using a given number of standard electrode locations and thereafter to use this pre-computed theoretical reference covariance matrix within the algorithm to automatically select, for example by use of electrode labels (e. g. Fp1, Fp2, etc.) present in the analyzed EEG signal, the electrode locations, respectively the parts of this pre-computed theoretical reference covariance matrix to be used for a specific EEG signal to be processed. Said given number of standard electrode locations may for example be formed by the above mentioned up to 343 electrodes, respectively the corresponding up to 343 electrode locations. Such theoretical reference covariance matrix may be generated with the OPENMEEG software from the ICBM152 head-model, using the Boundary Element Method (BEM). Alternative ways of constructing such theoretical reference covariance matrix (refCOV) by use of leadfield theory include for example using spherical shells or a Finite Element Method (FEM). The second option is to determine the locations of a given number of electrodes effectively used for a specific EEG signal to be processed and thereafter to compute a theoretical reference covariance matrix specifically adapted for this EEG signal to be analyzed. The second option may also be used for recordings with custom (i.e. non-standard) electrode locations. Preferably, within the second option, the BEM leadfield is re-computed, for example based on the ICBM152 head-model. In this context, it is preferred to use the so-called Fieldtrip Toolbox for re-computing the BEM leadfield, given that it is a computationally faster way of calculating the leadfield required for determining the theoretical reference covariance matrix (refCOV). However, the above mentioned alternatives of using spherical shells or the Finite Element Method (FEM) to construct the theoretical reference covariance matrix (refCOV) are feasible in the context of the second option, too. Both options allow to estimate a leadfield matrix, with dimensions [channels x sources], also known to a person skilled in the art as the forward model. Based thereupon, the theoretical spatial reference covariance matrix (refCOV), with dimensions [channels x channels], can be simply computed as: $refCOV = leadfield _ matrix * leadfield _ {matrix}^{T} .$

The validity of this source-space to electrode-space projection lies on key theoretical assumptions. First, it is assumed that the potential measured at a given electrode position is the resulting weighted contribution of all underlying dipolar sources. Second, it is assumed that the strength of each source is independent from other source parameters (e. g. the source weighting is independent from the frequency band emitted by the dipolar source). Under these conditions, it is possible to assume that there is a linear relation between the signal covariance from each electrode's time-domain signal and the summed as well as weighted covariance of the individual dipolar sources.

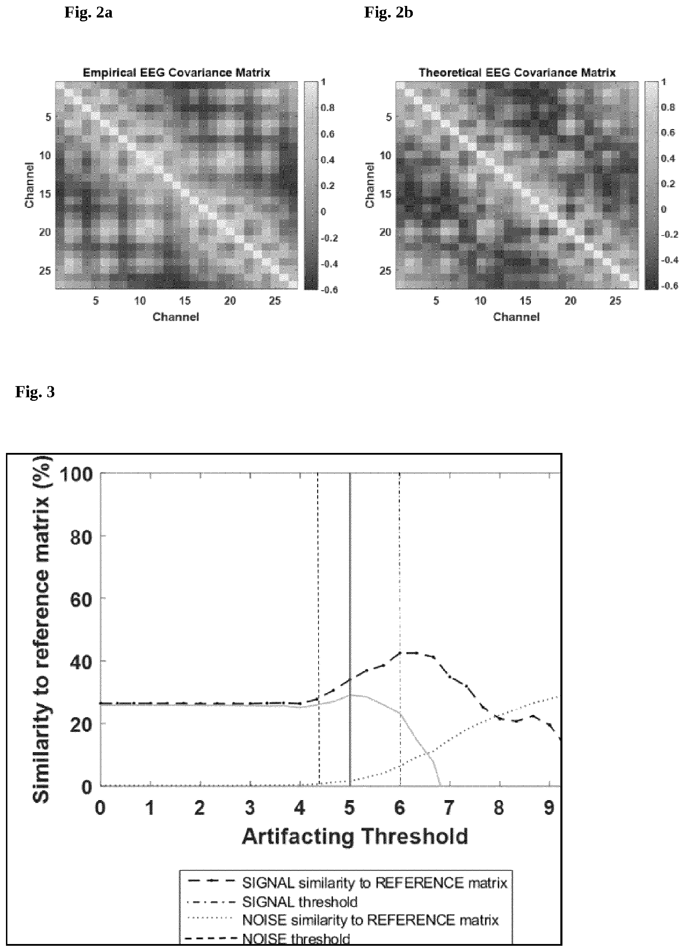

Basic empirical evidence supporting the aforementioned assumptions is provided in figure 2, where a covariance matrix extracted from clean EEG data is compared to a leadfield-generated covariance matrix. In particular, figures 2a and 2b schematically illustrate a comparison between an empirical spatial covariance matrix estimated from clean EEG resting-state data (figure 2a) and a theoretical spatial covariance matrix computed from the leadfield matrix of a Boundary Element Method (BEM) EEG forward model (figure 2b). Both matrices were normalised channel x channel, i. e. 0 mean with a standard deviation of 1.

### 2. Dividing into time intervals and generalized Eigenvalue decomposition

Threshold-based artifacts rejection methods of prior art rely on the assumption that artifacts are usually large and relatively rare events occurring in the signal, such that artifacts can be identified as appearing at the upper tail of the distribution of EEG/MEG signal components and be excluded based on a defined cutoff. This PCA-based approach is used in ASR and therefore subject to the orthogonality limitations mentioned above.

According to the present invention, in order to extract the magnitude of the EEG/MEG components to be removed from a given multi-channel signal, Generalized Eigenvalue Decomposition (GEVD) is used, which removes the orthogonality constraint and, briefly summarized in other terms, changes the meaning of large Eigenvalues from "components with a high variance" to "components with a high variance that deviate more than a pre-defined limit from the reference covariance matrix (refCOV)".". In layman's terms, the Eigenvalue decomposition proposed by the present invention can be seen as extracting components that describe the relation between both matrices of the matrix factorization provided by GEVD for said multi-channel signal, with the magnitude of the Eigenvalues reflecting the difference in variance and the direction of variance from the reference covariance matrix.

For the above mentioned example that the GEDAI algorithm is implemented as a Matlab EEGlab plugin, this may be summarised in one line of the corresponding Matlab code, with i being the index of the time interval, as "[generalized_eigenvaluesᵢ, generalized_eigenvectorsᵢ] = eig(dataCOVᵢ, refCOV);".

Mathematically, the matrix factorization by GEVD follows the linear equation $dataCOV ∗ V = Λ ∗ refCOV ∗ V ,$ where dataCOV is the data covariance matrix extracted from the EEG/MEG multi-channel signal in a single time interval, refCOV is the theoretical spatial reference covariance matrix determined at the previous step described in the preceeding chapter, A is the diagonal matrix containing the generalized Eigenvalues and V is the matrix containing the generalized Eigenvectors. According to the proposed GEDAI algorithm, the reference covariance matrix (refCOV) preferably is regularized before applying the GEVD by reducing all values by a percentage comprised in the range of 0,3 % to 10 %, and then adding a percentage comprised in the range of 0,3 % to 10 % of the mean of all Eigenvalues to the diagonal of the reference covariance matrix. This regularization procedure is used to improve numerical stability and handling of singular (i.e. rank deficient) matrices, as well as minimizing overfitting. Once the GEVD has been performed in all the time intervals of the multi-channel signal to be processed, the Eigenvalues extracted for each time interval can be pooled together to determine, based on their distribution, the ideal Eigenvalue cut-off point above which all components are considered to be artifactual, its Eigenvectors, respectively Eigenvalues, representing component topographies, respectively magnitudes.

### 3. Finding the threshold for artifactual components (SENSAI function)

Like mentioned above, for a given threshold T, the components associated with Eigenvalues above the threshold T are removed from a given empirical multi-channel signal for each time interval. This is done by (pseudo-)inverting the Eigenvector matrix V and removing, i. e. zeroing, the columns associated with the Eigenvalue above the threshold (same column number when the matrices are sorted in descending order). The new matrix M obtained this way contains vectors associated with the topographies of the non-artifactual parts of the EEG/MEG multi-channel signal in the time interval processed. The efficacy of such data cleaning procedure relies on the value of the threshold *T*, which in most prior art GEVD applications is usually provided by the user as an input. According to the present invention, the proposed GEDAI algorithm automatically sets the optimal threshold for Eigenvalue cutoff, without user input, by evaluating the improvement in the EEG/MEG output signal at multiple values of *T*, with said improvement referring to cosine similarity of the produced EEG/MEG output signal with the theoretical reference covariance matrix (refCOV). In particular, according to the present invention, said evaluation is done via the above mentioned, so-called SENSAI function (Signal & Noise Subspace Alignment Index) of the proposed GEDAI algorithm. The SENSAI function is schematically and exemplarily illustrated in figure 3 and measures the cosine similarity between the reference covariance matrix (refCOV) and either the retained parts (referred to as "signal" in figure 3) or the removed parts (referred to as "noise" in figure 3) of the EEG/MEG empirical multi-channel signal or both.

In the preferred embodiment shown as an example in figure 3, the SENSAI function of the proposed GEDAI algorithm computes, for each threshold value tested, a similarity index of the retained and removed parts of the EEG/MEG empirical multi-channel signal. The similarity index is calculated by taking the covariance matrix of the processed, i. e. cleaned/denoised, EEG/MEG multi-channel signal as well as of the reference covariance matrix (refCOV) and by separately performing on both a classical Eigenvalue decomposition, i. e. a decomposition equivalent to the one performed in PCA. Whilst in general all or a number within the range of 1 to 6 of the top Eigenvectors may be used for this purpose, in the example shown in figure 3 the principal angles between the top 3 components (i. e. the first 3 Eigenvectors sorted in descending order by Eigenvalue magnitude) of the reference covariance matrix (refCOV) and of the cleaned multi-channel signal subspaces are then estimated. Here, given two subspaces with their orthonormal bases *A* (from the reference covariance matrix (refCOV)) and *B* (from the cleaned multi-channel signal), the principal angles θᵢ between these subspaces can be computed as follows:
a. Compute a projection matrix C according to equation $C = {A}^{T} B .$
b. Perform Singular Value Decomposition (SVD) on the projection matrix *C* according to equation $C = {WΣQ}^{T} ,$ where W is related to the orthonormal basis of the subspace *A*, Q is related to the orthonormal basis of the subspace B, and Σ is a diagonal matrix containing the cosines of the principal angles between the two subspaces.
c. The principal angles θᵢ are obtained from the singular values σᵢ in the diagonal matrix Σ according to equation
   θ ᵢ=arccos(σᵢ) for i=1,2,3 .

Finally, the cosine of each principal angle is taken and multiplied with each other, yielding a so-called Signal Subspace Similarity Index (SSSI) according to equation $Signal subspace similarityindex = cos \left(θ1\right) ∗ cos \left(θ2\right) ∗ cos \left(θ3\right) ,$ where *θ*1, *θ*2 and *θ*3 are the principal angles between the top 3 Eigenvectors of the cleaned multi-channel signal and the top 3 Eigenvectors of the reference covariance matrix (refCOV). Similarly, a so-called Noise Subspace Similarity Index (NSSI) is also estimated between the top 3 Eigenvectors of the removed parts of the EEG/MEG empirical multi-channel signal, i. e. the noise data which is removed during signal processing from the multi-channel signal, and the reference covariance matrix (refCOV). By estimating the SSSI and NSSI through a broad test range of different threshold values, it becomes possible to optimize the tradeoff between removing and retaining too many artifactual components. For example, as visible in figure 3, increasing the threshold strength at first increases the similarity of the cleaned multi-channel signal with the reference covariance matrix (refCOV), as non-cerebral signal components are removed. Past a certain point however, the threshold becomes too aggressive and signal components corresponding to cerebral activity start to be removed from the EEG/MEG multi-channel signal, thus reducing the similarity of the cleaned multi-channel signal with the reference covariance matrix (refCOV). Similarly, by calculating the NSSI for the parts of the EEG/MEG multi-channel signal being removed during signal processing, it is possible to quantify how much of the multi-channel signal potentially corresponding to cerebral EEG/MEG activity gets discarded from the signal as noise throughout the test range of different threshold values.

The SENSAI function of the proposed GEDAI algorithm preferably calculates a tradeoff score, called the SENSAI score, by subtracting the subspace similarity index of the noise, i. e. the above mentioned Noise Subspace Similarity Index (NSSI), from the similarity index of the cleaned multi-channel signal, i. e. the above mentioned Signal Subspace Similarity Index (SSSI), or mathematically $SENSAI score = Signal subspace similarity index - Noise subspace similarity index .$

The optimal threshold for cleaning the data may then be found by maximising this score. The SENSAI function may also be applied by use of weights modifiying more or less the removal of parts of the multi-channel signal potentially corresponding to cerebral EEG/MEG activity, depending on the desired or required cleaning strength. For example, in the above equation, the NSSI can also be preceded by a linear multiplier, e. g. with values usually between 1 to 10, with a default of 3, to alter the weighting between the signal and noise subspaces. Smaller values remove more noise, but also risk removing more signal.

For example and as exemplarily illustrated in figure 3, the SENSAI function of the proposed GEDAI algorithm may sweep through different test threshold values T ranging from 0.2 to 9.8 in strength, for example in steps of 0.2. This range and step size was chosen arbitrarily as an example to provide an intuitive scale and is calculated according to equation $T 1 = \left(105-T\right) ∗ 100 .$

Thus, T1 ranges between 1.048 and 0.952. A Probability Integral Transform (PIT) fitting is then applied to the T1 threshold value to scale it based on the 0.95th percentile of the empirical cumulative distribution function of the Eigenvalues found in the EEG/MEG multi-channel signal. In other words, the SENSAI function of the proposed GEDAI algorithm ensures that the threshold is proportional to the threshold value at which the top 5% of the Eigenvalues are excluded and then sweeps from 95.2% to 104.5% of that value to find the optimal threshold. These values may be chosen differently and have been empirically determined, for the example illustrated in figure 3, during the code optimisation of the SENSAI function of the proposed GEDAI algorithm.

Figure 3 schematically illustrates the steps mentioned above, showing that, at each tested threshold value, the Signal Subspace Similarity Index (SSSI/dashed curve) tracks the similarity of the cleaned EEG/MEG multi-channel signal with the reference covariance matrix (refCOV), while the Noise Subspace Similarity Index (NSSI/dotted curve) expresses the similarity of the removed parts of the EEG/MEG multi-channel signal with the theoretical reference covariance matrix (refCOV). The SENSAI score (line curve) represents their absolute difference, where its maximum defines the optimum value for the threshold (vertical line) to be applied. For reference, the right dashed line is the maximum of the SSSI curve, while the left dashed line indicates the change point of the NSSI curve, i. e. when the similarity between the noise removed from the EEG/MEG multi-channel signal and the reference covariance matrix (refCOV) starts climbing.

In general, it should be noted that the specific values mentioned above in the context of the example illustrated in figure 3, for example the values for the test range of threshold values, the step size and other values related thereto, mainly serve to exemplarily illustrate this preferred embodiment and this specific case of application, but that it is possible to use other values. In particular, like mentioned above, it is possible in less preferred embodiments to apply said SENSAI function in a manner to evaluate either only the similarity between the reference covariance matrix (refCOV) and the retained parts of the EEG/MEG multi-channel signal, i. e. to evaluate only similarity of the output signal, or alternatively only the similarity between the reference covariance matrix (refCOV) and the removed parts of the EEG/MEG empirical multi-channel signal, i. e. to evaluate only similarity of the removed noise. In these embodiments as well as in different applications, it is possible to use different values.

### 4. Signal reconstruction

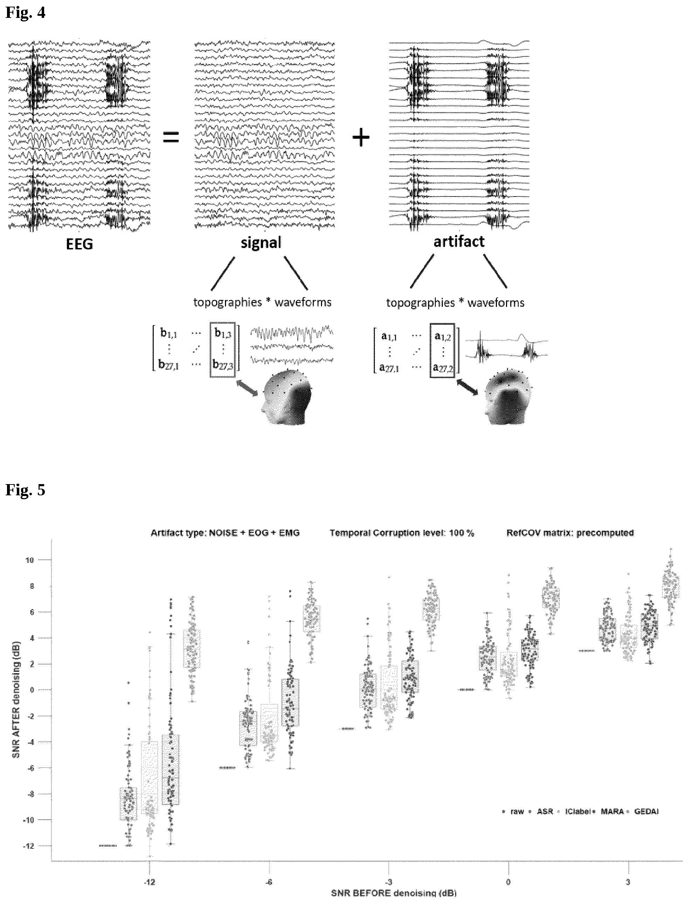

If the acquired empirical EEG/MEG multi-channel signal, to be processed by use of a method according to the present invention, has a length in time requiring that the step of extracting a data covariance matrix (dataCOV) from the acquired multi-channel signal is performed by dividing the signal, by use of adequate time intervals, into time intervals, as is the case in the preferred embodiment of the method described above, the step of producing the output signal of the method, i. e. reconstruction of the cleaned EEG/MEG multi-channel signal, has to be done in each of said time intervals, too. First, the vectors (columns) associated with Eigenvalues smaller than the applied threshold are removed from the matrix *V* containing the Eigenvectors of the GEVD decomposition. This creates a spatial filter selecting only the components to be removed. The same process is applied to the pseudo-inverse of the Eigenvector matrix to obtain individual activation maps, also referred to as "topographies", of the artifactual components. The principle of linearly decomposing the EEG/MEG multi-channel signal is illustrated in figure 4 which symbolically shows that an EEG/MEG multi-channel signal may be considered to be a linear summation of electrical activities from a "brain signal" subspace and of electrical activities from an "artifact signal" subspace containing different types of non-cerebral noise or artifacts. This mixture may be "unmixed" by linear decomposition techniques (e. g. PCA, ICA, GEVD) into separate components with individual source locations ("topographies) and respective time-courses ("waveforms"). The GEDAI algorithm proposed by the present invention combines theoretical knowledge of the clean "brain signal" subspace (i. e. its spatial covariance) with GEVD to further separate components belonging to the "artifact signal" subspace from those of the "brain signal" subspace (i. e. by sorting components based on their Eigenvalue magnitude). Based thereupon, the next step of producing the output signal of the method according to the present invention consists in obtaining the individual time courses, also referred to as "waveforms", of the artifactual components by multiplying the transpose of this spatial filter matrix with the time course of the EEG/MEG multi-channel signal. Finally, by multiplying the activation map matrix with the artifactual components' waveforms, the time courses of the artifacts are obtained, in electrodes space. By subtracting this artifacts signal from the acquired EEG/MEG multi-channel signal, the output signal, i. e. the cleaned EEG/MEG multi-channel signal, is obtained.

### 5. Frequency-specific GEDAI

To further optimize the denoising capacity of a method according to the present invention, it is possible to include into the method the option of running the cleaning pipeline of the proposed GEDAI algorithm on spectrally decomposed EEG/MEG multi-channel signals. The interest of this optional supplementary step comes from the fact that brain signals and/or artifact distributions may differ between EEG/MEG frequency bands, which may have a frequency range from delta (frequency between 1 Hz and 4 Hz) to gamma (frequency between 30 Hz and 150 Hz) bands. Thus, frequency-specific thresholds might be interesting, or even required, in certain applications, to ensure that high-frequency artefacts (e. g. beta) are not rejected using the same Eigenvalue threshold estimated for lower frequency bands, e. g. for the delta band (frequency between 13 Hz and 30 Hz), or vice versa. As such, an embodiment of the proposed GEDAI algorithm using frequency-specific thresholds may be provided as a Matlab EEGlab plugin and starts by first running as classical broadband algorithm, i. e. using only one broadband frequency band (e. g. 0.5 Hz - 45 Hz), with subsequent second rounds on discrete wavelet decomposed signals filtered in a number of dyadic frequency bands, for example in 5 to 15 dyadic frequency bands, preferably in 10 dyadic frequency bands using the so-called "Haar" wavelet, which has excellent time localisation. For example, for an EEG signal sampled at 256 Hz, the wavelet bands produced by the discrete wavelet transform (DWT) are as follows: 64-128 Hz (band 1), 32-64 Hz (band 2), 16-32 Hz (band 3), 8-16 Hz (band 4), 4-8 Hz (band 5), 2-4 Hz (band 6), 1-2 Hz (band 6), 0.5-1 Hz (band 8), 0.25-0.5 Hz (band 9), 0.125-0.25 Hz (band 10). The optimal threshold to be applied by the above described SENSAI function of the proposed GEDAI algorithm is thus estimated separately for each frequency band and the output signal is reconstructed by adding all the resulting signals together after cleaning.

### 6. Data overlap

Given that the preferred embodiment of the proposed GEDAI algorithm is a denoising/cleaning method based on dividing the acquired multi-channel signal into time intervals, the step of producing the output signal of the method, i. e. reconstruction of the cleaned EEG/MEG multi-channel signal, by concatenating denoised/cleaned parts of the multi-channel signal divided into time intervals leads, in the preferred embodiment, to signal discontinuities at the edges of the time intervals. To compensate for this problem, the proposed GEDAI algorithm processes the acquired multi-channel signals by use of overlapping windows with respect to the time intervals, for example with 50% overlapping windows, that are then cosine-weighted during reconstruction to remove discontinuities. The principle of using overlapping windows being already known in prior art cleaning algorithms, such as ASR, it isn't required to indicate further details on this topic.

### Verification and Benchmarking

In order to verify and benchmark a method for signal processing for removal of artifacts from EEG/MEG signals according to the present invention, its performance is compared in the following to several known implementations of spatial filtering methods, in particular to the prior art methods ASR [7], ICLabel [21] and MARA [22]. The first method is known for its computational speed. The latter two methods are considered in prior art as the golden standard of EEG/MEG de-artifacting, because of yielding high quality results. The benchmarking simulations were performed by using a combination of two publicly-available EEG datasets [23, 24] containing specific artifacts classes, e. g. EOG, EMG, movement. The first dataset was recorded in healthy subjects using multi-channel recordings containing clean and artefact-related EEG [23], while the second dataset was constructed using recordings from individual EOG/EMG electrodes [24].

For clean EEG signal generation during the benchmarking simulations, experimentally acquired recordings of clean EEG data in said first dataset [23] were used, where 10 separate subjects were selected from this dataset in the 'eyes-closed' resting condition with duration of 100 seconds. These recordings were visually inspected for the presence of any artifacts, and any samples that were suspected of contamination were wholesale rejected, i. e. removed across all channels. For every subject, clean EEG segments of at least 2 seconds were concatenated and low-pass filtered at 60 Hz. Each output dataset had a duration of 60 seconds, and consisted of 27 channels with a sampling frequency of 200 Hz. Please see Figure 4 for an example of a clean EEG "signal" that was used. Likewise, for the artifact signal generation during the benchmarking simulations, Since scalp EEG recordings of extra-cerebral artifacts inevitably contain brain signal (preventing the establishment of a pure artifact ground-truth), empirical recordings of sensor data directly overlying the eyes (EOG, electro-oculogram) or facial-muscles (EMG, electromyogram) were used from said second dataset [24]. Here, as schematically shown in figure 4, the EOG/EMG single time-series ("waveforms") were multiplied by relevant spatial patterns ("topographies"). In order for these topographies to be consistent with EOG/EMG generation, extended Infomax-ICA was performed separately on multichannel EOG/EMG artifact recordings from said first dataset [23]. Then, independent components of EOG/EMG were visually identified by an expert and only their topographic information was retained. Finally, as described above, these EOG/EMG topographies were respectively multiplied by EOG/EMG single time-series, with time intervals having a length of 2 seconds, to construct multi-channel datasets containing only artifacts. EOG signals were band-pass filtered between 0.3 and 10 Hz and then re-sampled to 200 Hz. EMG signals were band-pass filtered between 1 to 120 Hz, notched at the powerline frequency of 50 Hz and then resampled to 200 Hz. Each output dataset had a duration of 60 seconds and consisted of 27 channels with a sampling frequency of 200 Hz. Figure 4, neyt to schematically illustrating the principle of linearly decomposing an EEG/MEG multi-channel signal, also shows an example of an EMG "artifact" that was used. Datasets with noise artifacts were identical in size and were generated to contain the following types of natural (e. g. based on empirical recordings in said first dataset [23]) and artificial (i. e. Matlab simulated) noise sources: movement artifact (empirical), electrode pop (empirical), bad channels (simulated), step artifacts (simulated), and spatiotemporally non-stationary noise (simulated).

In order to challenge the denoising algorithms with more complex scenarios of artifact mixtures, the baseline EOG, EMG, and/or NOISE datasets were z-score normalised and linearly combined with each other to produce two additional artifact categories: "EMG + EOG", and "NOISE + EMG + EOG". In total, 10 separate artifact datasets were generated for each of the following 5 artifact categories:
1) EMG (electromyogram) i.e. muscle activity
2) EOG (electro-oculogram) i.e. eye blinks and/or movement
3) EOG + EMG
4) NOISE (channel or background noise)
5) NOISE + EMG + EOG .

For the final step of mixed "EEG + artifact" signal generation, and in accordance with the model illustrated in figure 4, each of the 10 clean recordings was pair-wise "mixed", through linear summation, with each of 10 datasets from a particular artifact category. This resulted in 100 contaminated datasets within each one of the 5 artifact categories (EMG, EOG, EOG + EMG). Hence, the total benchmarking sample comprised of 500 datasets, each with 27 channels and a length of 60 seconds, sampled at 200 Hz (i. e. each data matrix: 27 channels x 12,000 samples). Simulations were run based on the model illustrated in figure 4. Here, the clean EEG recording acted as the ground truth signal, which was to be recovered by the denoising algorithms operating blindly on the contaminated (i. e. mixed) datasets.

In terms of reference denoising methods, the following three prior art methods were used to benchmark a method according to the present invention as compared:
First, a comparison was done with respect to a PCA based method, namely to ASR [7], as it is a fast, real-time capable method for EEG cleaning.

The second method compared to a method according to the present invention is ICLabel [21]. This ICA-based technique can automate artifact rejection by evaluating ICA topographies with a machine learning based classifier trained on ICA topographies from over 6,000 datasets.

Lastly, a method according to the present invention is compared to MARA (Multiple Artifact Rejection Algorithm, [22]). This technique is also ICA-based, but contrary to ICLabel the machine learning classifier was trained on a large database of expert-labeled artifactual topographies.

Each algorithm was run with its default parameter settings, which was done to test each algorithm's adaptation to a wide range of noise scenarios.

Concerning the results of the benchmarking, figure 5 shows the results of the benchmarking comparison in terms of reconstruction accuracy specifically for datasets with 100% temporal corruption (i. e. artifacts present in every 2 second time interval) and multiple concomitant artifacts (i. e. NOISE + EOG + EMG). In particular, every data point represents a dataset's SNR (signal to noise ratio) after denoising (along the y-axis), across different baseline noise levels before denoising (along the x-axis). In this comparison, a higher SNR value on the y-axis represents a better reconstruction accuracy, where ${SNR}_{before} = 10 ∗ log 10 \left(original_signal_power/original_noise_power\right)$ ${SNR}_{after} = 10 ∗ log 10 \left(original_signal_power/residual_noise_power\right) .$

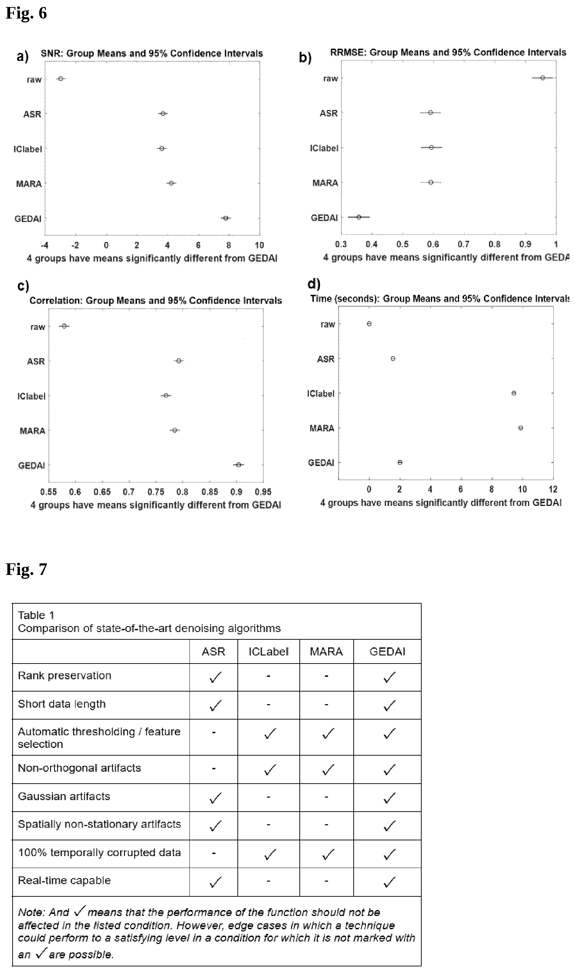

Figures 6a to 6d show a summary of the results of this comparison by indicating the aggregate means and 95% confidence intervals across across all 500 datasets and therefore across all artifact types (EMG, EOG, EMG + EOG, NOISE, EMG + EOG + NOISE). In particular, this is indicated in figure 6a for SNR (the signal to noise ratio after denoising), where a higher value represents a better result, and in figure 6b for RRMSE (relative root mean square error), where a lower value represents a better result. Figure 6c shows the results of this comparison in terms of reconstruction accuracy, in particular by using the correlation coefficient (√R). In this comparison, a higher value represents a better reconstruction accuracy. Figure 6d shows the results of this comparison in terms of average processing time, in seconds per dataset. All datasets were of an identical size of 27 channels x 12000 samples. In this comparison, a lower value represents better performance. Figure 7 is a table summarizing the results of this comparison in terms of specific characteristics of the investigated denoising techniques.

Based on these comparisons, it is possible to qualify a method for signal processing for removal of artifacts from EEG/MEG signals according to the present invention as surpassing prior art methods representing the current golden standard both in terms of speed and accuracy.

In particular, this comparison demonstrates that a method for signal processing for removal of artifacts from EEG/MEG signals according to the present invention represents a significant advancement in EEG artifact removal, offering a compelling combination of speed, accuracy, and full automation, particularly in challenging, multi-artifact contaminated EEG recordings. As shown in figure 5, the proposed GEDAI algorithm demonstrates superior performance compared to all investigated prior art methods when considering severely corrupted EEG signals containing several concomitant contamination sources (EMG + EOG + NOISE). The embodiment of a signal processing method according to the present invention called GEDAI in figures 6a to 6d, also always performed better than, or on par with, the prior art algorithms in simulations when aggregating across all types of artifact (i.e. EMG, EOG, EMG + EMG, NOISE, EOG + EMG + NOISE +). As shown in figure 6d, the average processing time per dataset for the proposed GEDAI algorithm was around 2 seconds, which was comparable to the average speed of the ASR cleaning algorithm (1.7 seconds), which is already considered as being real-time capable.

In terms of accuracy, measured by evaluating the similarity of the output signal, i. e. the cleaned EEG/MEG multi-channel signal, with the "ground-truth" empirical and synthetic datasets, the signal processing method according to the present invention surpassed the investigated prior art methods most likely because of its greater flexibility in artifact definition, as it is not constrained by orthogonality or non-gaussianity. This advantage is particularly marked for datasets that include the "noise" condition, for which artefacts were in many cases spatially non-stationary. However, results indicate a trend for higher performance of a signal processing method according to the present invention in datasets where multiple other concomitant artifacts (i. e. NOISE + EOG + EMG) were present. The edge of the latter method is achieved by allowing for frequency-band specific thresholding, as this embodiment of the method according to the present invention can better target artifacts that are prominent in specific frequency ranges, e. g. muscle artifacts in higher frequencies, eye movements in lower frequencies, while preserving the integrity of neural oscillations in other bands. Additionally, this embodiment preferably forms an iterative signal processing method that starts by executing the signal processing method according to the present invention using a single threshold before starting the frequency-specific decomposition.

Regarding the algorithmic speed, the comparable processing time of the proposed GEDAI algorithm as compared to ASR (for EEG data with fewer than 30 channels) allows for online applications, given that the wavelet decomposition across frequency bands can be computed in parallel, i.e. simultaneously across multiple CPU and/or GPU cores. Moreover, the embodiment of a signal processing method according to the present invention using frequency-specific thresholds performed approximately 4 times faster than IClabel, the faster one of the two investigated ICA prior art methods. It should be noted that the speed of a signal processing method according to the present invention is lower for datasets with more than 30 electrodes, as electrode number increases GEVD processing time cubically, with time complexity of O(n³).

In any case, a signal processing method according to the present invention is fully automated and requires no operator expertise or input. While these characteristics are partially present also in other prior art methods investigated during the benchmarking, a signal processing method according to the present invention can achieve automation even without curated calibration data. This latter characteristic is of particular importance when the operator doesn't have enough knowledge to ensure that clean portions of data are present in the recording, as well as for fully corrupted data, e. g. in case of dry electrodes recordings.

Finally, it should be noted that a signal processing method according to the present invention intrinsically has to rely on multi-channel data to construct a reference covariance matrix which is essential for the EEG/MEG de-artifacting process according to the present invention. This means that, much like in prior art methods like ICA and ASR, the maximum number of artifactual components that can be removed from any time interval is limited to the number of EEG/MEG recording channels. Therefore, a signal processing method according to the present invention cannot be used on single-electrode EEG/MEG setups. However, the potential of a signal processing method according to the present invention for improving the quality of EEG/MEG multi-channel signals from dry electrode systems, which are often favoured due to their speed and ease of use, could incentivize the switch from single-channel to multi-channel setups, which allows to capture distributed brain activity.

The present invention is also related to a computer readable medium storing a computer program which causes a computer to implement the method according to present invention when the program is executed by the computer as well as to a device for acquiring a multi-channel signal by electro-encephalography (EEG) and/or magnetoencephalography (MEG) and/or for processing a multi-channel signal acquired by electroencephalography (EEG) and/or magnetoencephalography (MEG), the device comprising at least an input/output unit, a storage unit allowing to store acquired multi-channel signals, a processing unit allowing to process acquired multi-channel signals, the device being equipped with means adapted to perform the method for removal of artifacts from EEG/MEG multi-channel signals according to the present invention. In particular, such device may consist of a conventional EEG/MEG apparatus, with its input/output unit comprising a set of electrodes adapted to acquire a EEG/MEG multi-channel signal and its processing unit being modified such as to allow for signal processing according to the method of the present invention.

### Applications

The signal processing method according to the present invention, a fully unsupervised algorithm for removing extra-cerebral artifacts and noise from heavily contaminated magnetoencephalography/electroencephalography (M/EEG) recordings, leverages a generalized Eigenvalue decomposition (GEVD) to identify and remove noisy components that originate outside of the brain based on the EEG/MEG forward model. The theoretical nature of the model used in the method according to the present invention for the reference covariance matrix offers unique improvements over existing approaches, including 1) denoising of spatially non-stationary artifacts from highly contaminated recordings without any "clean" reference data, 2) unified correction of artifactual time intervals and bad channels, 3) adaptive thresholding based on noise level and 4) objective estimation of EEG/MEG denoising quality via the signal and noise subspace alignment index (SENSAI). When evaluated against simulated and empirical EEG data contaminated with realistic artifacts, for example eye blinks, muscle activity or environmental noise, the method according to the present invention globally outperformed established denoising techniques like independent component analysis (ICA) and artifact subspace reconstruction (ASR), demonstrating large improvements in challenging scenarios with limited clean data and/or complex artifact mixtures. Therefore, a method according to the present invention may be applied in 1) low-quality mobile and/or dry electrode recordings (with up to 100% temporal contamination), 2) MEG denoising (given the shared EEG/MEG forward mode), and 3) real-time processing, for example for brain-computer interfaces (BCIs) due to GEVD's rapid computations.

In general, EEG/MEG denoising is an essential pre-processing step in all settings that involve recordings of raw EEG/MEG signals. This is because the denoised EEG/MEG signal should reflect as closely as possible the underlying electrical (i.e. neuronal) brain activity, and not that of extra-cerebrally generated artifacts. EEG/MEG reflect artifacts include other physiological activity, e. g. muscle and eye movement, and/or environmental noise, e. g. electrode contact noise.

Therefore, a method according to the present invention may be included in many kinds of software and/or devices which includes EEG/MEG denoising. Such software/devices may be used for example in the context of medical applications such as
- Detection of Epilepsy and Seizure Disorders:
   EEG/MEG is a primary tool for detecting abnormal brain activity associated with seizures. It helps identify the type of seizures and localize their origin in the brain, which directly informs surgical or pharmacological treatments.
- Detection of Brain Disorders:
   EEG/MEG can assist in detecting various neurological conditions, including brain tumors, brain damage from head injuries, encephalitis (brain inflammation), stroke, dementia (e. g. Alzheimer's disease), sleep disorders (e. g. narcolepsy)
- Monitoring Brain Activity in Coma and Surgery:
   EEG/MEG is used to monitor brain function in patients in comas, helping to assess brain damage and determine brain death. It's also used in Intensive Care Units (ICU) and during brain surgery to monitor brain activity in order to ensure patient safety.
- Sleep Studies:
   EEG/MEG is a crucial component of polysomnography, a comprehensive sleep study used to diagnose sleep disorders.
- Medical Research:
   EEG/MEG is widely used in neuroscience research to study brain function, cognition, and various neurological and psychiatric conditions.
   Such software/devices may also be used for example in the context of commercial applications such as
- Brain-Computer Interfaces (BCIs):
   EEG/MEG is used in BCIs to enable communication and control using brain signals. Applications include assistive technology for people with disabilities, e.g. controlling wheelchairs, computers, or prosthetic devices.
- Cognitive Training and Performance Enhancement:
   EEG/MEG-based neurofeedback is used to train individuals to regulate their brain activity, potentially improving attention, memory, and other cognitive functions.
- Entertainment and Gaming:
   EEG/MEG is being incorporated into gaming and entertainment applications to create immersive experiences and provide personalized feedback.
- Wellness and Stress Management:
   Portable EEG/MEG devices are being developed for personal use to monitor stress levels and promote relaxation through neurofeedback.

A method, a computer program, and/or a device according to the present invention thus may be applied in a wide variety of fields.

### Advantages

In light of the above description of the structure and of the operating mode of a method according to the present invention for removal of artifacts from EEG/MEG signals using GEVD, it is clear that the method has several advantages, in particular due to use of said GEDAI algorithm. First, this method can disentangle non-orthogonal components in the EEG/MEG signal, which would not be possible for prior art techniques like ASR. Secondly, by focusing on defining "clean" EEG/MEG, the GEDAI algorithm is not limited to any particular artifact type or definition, e. g. to non-gaussianity in prior art techniques like ICA, such that a method according to the present invention has increased versatility and noise-robustness. Third, the GEDAI algorithm projects out contamination components instead of rejecting bad time intervals, thereby preserving all available temporal data. Fourth, this algorithm is computationally light and real-time capable, thus warranting its use for brain-computer interface (BCI) applications. Fifth, by relying on a theoretically defined reference covariance matrix (refCOV), the GEDAI algorithm is a good candidate for EEG/MEG recordings with dry-electrodes or at-home EEG/MEG applications, where a clean data sample might not be available for defining a reference covariance matrix in sufficiently precise manner. Sixth, owing to its automatic design, in particular with regard to automatically setting a threshold for Eigenvalue cutoff, the GEDAI algorithm is suitable for use on big data applications, such as normative modeling.

The following list enumerates all references which are cited in the above description:
[1] Thulasidas, M., Guan, C., Ranganatha, S., Wu, J. K., Zhu, X., & Xu, W. (2004). Effect of ocular artifact removal in brain computer interface accuracy. The 26th Annual International Conference of the IEEE Engineering in Medicine and Biology Society, 2, 4385-4388. https://doi.org/10.1109/IEMBS.2004.1404220
[2] Huster, R. J., Mokom, Z. N., Enriquez-Geppert, S., & Herrmann, C. S. (2014). Brain-computer interfaces for EEG neurofeedback: Peculiarities and solutions. International Journal of Psychophysiology, 91(1), 36-45. https://doi.org/10.1016/j.ijpsycho.2013.08.011
[3] Jung, T. P., Makeig, S., Humphries, C., Lee, T. W., McKeown, M. J., Iragui, V., & Sejnowski, T. J. (2000). Removing electroencephalographic artifacts by blind source separation. Psychophysiology, 37(2), 163-178
[4] Olbrich, S., Jödicke, J., Sander, C., Himmerich, H., & Hegerl, U. (2011). ICA-based muscle artefact correction of EEG data: What is muscle and what is brain?: Comment on McMenamin et al. Neurolmage, 54(1), 1-3. https://doi.org/10.1016/j.neuroimage.2010.04.256
[5] Frølich, L., Andersen, T. S., & Mørup, M. (2015). Classification of independent components of EEG into multiple artifact classes. Psychophysiology, 52(1), 32-45. https://doi.org/10.1111/psyp.12290
[6] Parra, L., & Sajda, P. (2003). Blind Source Separation via Generalized Eigenvalue Decomposition. Journal of Machine Learning Research, 4, 1261-1269.
[7] Kothe, C. A. E., & Jung, T.-P. (2015). Artifact removal techniques with signal reconstruction (World Intellectual Property Organization, Patent Appl. No. WO2015/047462A9 and US2016/0113587A1). https://patents.google.com/patent/WO2015047462A9/en
[8] de Cheveigné, A., & Parra, L. C. (2014). Joint decorrelation, a versatile tool for multichannel data analysis. Neurolmage, 98, 487-505. https://doi.org/10.1016/j.neuroimage.2014.05.068
[9] Cohen, M. X. (2022). A tutorial on generalized eigendecomposition for denoising, contrast enhancement, and dimension reduction in multichannel electrophysiology.
[10] Gouy-Pailler, C., Sameni, R., Congedo, M., & Jutten, C. (2009). Iterative Subspace Decomposition for Ocular Artifact Removal from EEG Recordings. In T. Adali, C. Jutten, J. M. T. Romano, & A. K. Barros (Éds.), Independent Component Analysis and Signal Separation (Vol. 5441, p. 419-426). Springer Berlin Heidelberg. https://doi.org/10.1007/978-3-642-00599-2_53
[11] Haslacher, D., Nasr, K., Robinson, S. E., Braun, C., & Soekadar, S. R. (2021). Stimulation artifact source separation (SASS) for assessing electric brain oscillations during transcranial alternating current stimulation (tACS). Neurolmage, 228, 117571. https://doi.org/10.1016/j.neuroimage.2020.117571
[12] Menrath, D., Woller, J. P., & Gharabaghi, A. (2024). Real-Time Spatiotemporal Filtering for Artifact-Free EEG during Electrical Neurostimulation (p. 2024.11.06.622272). bioRxiv. https://doi.org/10.1101/2024.11.06.622272
[13] Woller, J. P., Menrath, D., & Gharabaghi, A. (2024). EEG denoising during transcutaneous auricular vagus nerve stimulation across simulated, phantom and human data (p. 2024.05.13.593884). bioRxiv. https://doi.org/10.1101/2024.05.13.593884
[14] Somers, B., Francart, T., & Bertrand, A. (2018). A generic EEG artifact removal algorithm based on the multi-channel Wiener filter. Journal of Neural Engineering, 15(3), 036007. https://doi.org/10.1088/1741-2552/aaac92
[15] Samadi, S., Amini, L., Soltanian-Zadeh, H., & Jutten, C. (2011). Identification of brain regions involved in epilepsy using common spatial pattern. 2011 IEEE Statistical Signal Processing Workshop (SSP), 825-828. https://doi.org/10.1109/SSP.2011.5967833
[16] Blankertz, B., Kawanabe, M., Tomioka, R., Hohlefeld, F., Müller, K.-R., & Nikulin, V. (2007). Invariant Common Spatial Patterns: Alleviating Nonstationarities in Brain-Computer Interfacing. Advances in Neural Information Processing Systems, 20. https://proceedings.neurips.cc/paper/2007/hash/6aab1270668d8cac7cef2566a1c5 f569-Abstract.html
[17] Lotte, F., & Guan, C. (2010). Spatially Regularized Common Spatial Patterns for EEG Classification. 2010 20th International Conference on Pattern Recognition, 3712-3715. https://doi.org/10.1109/ICPR.2010.904
[18] Reuderink, B., & Poel, M. (2008). Robustness of the Common Spatial Patterns algorithm in the BCI-pipeline. https://research.utwente.nl/en/publications/robustness-of-the-common-spatial-patterns-algorithm-in-the-bci-pi
[19] Chang, C.-Y., Hsu, S.-H., Pion-Tonachini, L., & Jung, T.-P. (2018). Evaluation of Artifact Subspace Reconstruction for Automatic EEG Artifact Removal. 2018 40th Annual International Conference of the IEEE Engineering in Medicine and Biology Society (EMBC), 1242-1245. https://doi.org/10.1109/EMBC.2018.8512547
[20] Nolan, H., Whelan, R., & Reilly, R. B. (2010). FASTER: Fully Automated Statistical Thresholding for EEG artifact Rejection. Journal of neuroscience methods, 192(1), 152-162. https://doi.org/10.1016/j.jneumeth.2010.07.015
[21] Pion-Tonachini, L., Kreutz-Delgado, K., & Makeig, S. (2019). ICLabel: An automated electroencephalographic independent component classifier, dataset, and website. Neurolmage, 198, 181-197. https://doi.org/10.1016/j.neuroimage.2019.05.026
[22] Winkler, I., Brandl, S., Horn, F., Waldburger, E., Allefeld, C., & Tangermann, M. (2014). Robust artifactual independent component classification for BCI practitioners. Journal of Neural Engineering, 11(3), 035013. https://doi.org/10.1088/1741-2560/11/3/035013
[23] https://github.com/stefan-ehrlich/dataset-automaticArtifactRemoval
[24] https://github.com/ncclabsustech/EEGdenoiseNet

### List of reference signs

| Nr. | Element |
|---|---|
| | |
| 1 | Method step of obtaining a EEG/MEG multi-channel signal |
| 2 | Method step of providing a matrix factorization of said multi-channel signal |
| 2.1 | Method step of determining a reference covariance matrix (refCOV) |
| 2.1a | Method step of computing a theoretical spatial reference covariance matrix (refCOV) by use of a given number of standard electrode locations |
| 2.1b | Method step of determining locations of a number of electrodes effectively used and computing a theoretical spatial reference covariance matrix (refCOV) specifically adapted for the signal to be processed |
| 2.2 | Method step of dividing the acquired multi-channel signal to be processed, by use of adequate time intervals, into time intervals |
| 2.3 | Method step of extracting for each time interval a data covariance matrix (dataCOV) |
| 2.4 | Method step of producing a generalized Eigenvalue decomposition of each data covariance matrix (dataCOV) |
| 3 | Method step of producing an output signal corresponding to said multi-channel signal |
| 3.1 | Method step of automatically setting a threshold for Eigenvalue cutoff |

## Claims

1. Signal processing method for removal of artifacts from electroencephalography (EEG) and/or magnetoencephalography (MEG) signals by use of Generalized Eigenvalue Decomposition (GEVD), the method comprising the steps of
- obtaining (1) a multi-channel signal acquired by electroencephalography (EEG) and/or magnetoencephalography (MEG),
- providing (2) a matrix factorization of said multi-channel signal by applying a generalized Eigenvalue decomposition to the signal, providing said matrix factorization of the multi-channel signal comprising the substeps of
° determining (2.1) a reference covariance matrix (refCOV) to be used as an input value to the Eigenvalue decomposition of said multi-channel signal,
° extracting (2.3) a data covariance matrix (dataCOV) from the acquired empirical multi-channel signal and
° producing (2.4) a generalized Eigenvalue decomposition of said data covariance matrix (dataCOV) corresponding to said multi-channel signal based on said reference covariance matrix (refCOV),
- producing (3), by use of said matrix factorization of the multi-channel signal, an output signal corresponding to said multi-channel signal with artifacts in the signal being removed by applying an Eigenvalue cutoff and non-artifact parts of the signal being retained,
**characterised by the fact that** the step of
- determining the reference covariance matrix (refCOV) is performed, without use of empirical data of the EEG/MEG multi-channel signal, by estimating a theoretical spatial reference covariance matrix (refCOV) based on principles of EEG/MEG signal generation, utilizing electrode locations and leadfield theory.

2. Method according to the preceding claim, **characterised by the fact that** estimating (2.1a) the theoretical spatial reference covariance matrix (refCOV) is done by use of a given number of standard electrode locations and of a pre-computed leadfield model corresponding to these standard electrode locations.

3. Method according to claim 1, **characterised by the fact that** estimating (2.1b) the theoretical spatial reference covariance matrix (refCOV) is done by determining the locations of a given number of electrodes effectively used for the specific multi-channel signal to be processed and thereafter computing, by use of a leadfield model specifically computed for these electrode locations, a theoretical reference covariance matrix (refCOV) specifically adapted for this multi-channel signal to be processed.

4. Method according to one of the preceding claims 2 to 3, **characterised by the fact that** the pre-computed and/or the specifically computed leadfield model is realized by a leadfield matrix constructed by use of a Boundary Element Method (BEM) EEG/MEG forward model, of spherical shells or of a Finite Element Method (FEM).

5. Method according to one of the preceding claims, **characterised by the fact that,** before applying the generalized Eigenvalue decomposition to said data covariance matrix (dataCOV), the reference covariance matrix (refCOV) is regularized by reducing all values by a percentage comprised in the range of 0,3 % to 10 % and then adding a percentage comprised in the range of 0,3 % to 10 % of the mean of all Eigenvalues to the diagonal of the reference covariance matrix, in order to improve numerical stability and handling of singular matrices as well as to minimize overfitting.

6. Method according to one of the preceding claims, **characterised by the fact that** the step of extracting the data covariance matrix (dataCOV) from the acquired empirical multi-channel signal is performed by dividing (2.2) the acquired multi-channel signal to be processed, by use of adequate time intervals, into time intervals and, if desired/required, recording the divided multi-channel signal.

7. Method according to the preceding claim, **characterised by the fact that** the step of producing the generalized Eigenvalue decomposition of said data covariance matrix (dataCOV) is performed for each data covariance matrix (dataCOV) in each time interval based, across all time intervals, on said reference covariance matrix (refCOV).

8. Method according to one of the preceding claims, **characterised by the fact that** the step of producing the output signal is performed by automatically setting (3.1) a threshold for Eigenvalue cutoff by iteratively comparing the effect of different threshold values on subspace similarity of the produced EEG/MEG output signal with the reference covariance matrix (refCOV).

9. Method according to the preceding claim, **characterised by the fact that** said comparison is performed by computing the subspace similarities between the Eigenvectors of the processed multi-channel signal and the Eigenvectors of the reference covariance matrix (refCOV), preferably between a number in the range of 1 to 6 of the top Eigenvectors of the processed multi-channel signal and a number in the range of 1 to 6 of the top Eigenvectors of the reference covariance matrix (refCOV).

10. Method according to one of the preceding claims 8 to 9, **characterised by the fact that** said iterative comparison of the effect of different threshold values in order to automatically set the threshold for Eigenvalue cutoff is performed on the parts of the signal being retained and/or on the parts of the signal being removed.

11. Method according to one of the preceding claims 6 to 10, **characterised by the fact that** the step of producing the output signal is performed by use of the acquired multi-channel signal divided into said time intervals.

12. Method according to one of the preceding claims 8 to 10, **characterised by the fact that** the step of producing the output signal is performed by using frequency-specific thresholds for Eigenvalue cutoff, each frequency-specific threshold being determined specifically for given frequency band of the acquired multi-channel signal.

13. Signal processing method for removal of artifacts from electroencephalography (EEG) and/or magnetoencephalography (MEG) signals by use of Generalized Eigenvalue Decomposition (GEVD), the method comprising the steps of
- obtaining (1) a multi-channel signal acquired by electroencephalography (EEG) and/or magnetoencephalography (MEG),
- providing (2) a matrix factorization of said multi-channel signal by applying a generalized Eigenvalue decomposition to the signal, providing said matrix factorization of the multi-channel signal comprising the substeps of
° determining (2.1) a reference covariance matrix (refCOV) to be used as an input value to the Eigenvalue decomposition of said multi-channel signal,
° extracting (2.3) a data covariance matrix (dataCOV) from the acquired empirical multi-channel signal and
° producing (2.4) a generalized Eigenvalue decomposition of said data covariance matrix (dataCOV) corresponding to said multi-channel signal based on said reference covariance matrix (refCOV),
- producing (3), by use of said matrix factorization of the multi-channel signal, an output signal corresponding to said multi-channel signal with artifacts in the signal being removed by applying an Eigenvalue cutoff and non-artifact parts of the signal being retained,
**characterised by the fact that** the step of producing the output signal is performed by automatically setting (3.1) a threshold for Eigenvalue cutoff by iteratively comparing the effect of different threshold values on subspace similarity of the produced EEG/MEG output signal with the reference covariance matrix (refCOV).

14. Computer readable medium storing a computer program which causes a computer to implement the method according to one of the preceding claims when the program is executed by the computer.

15. Device for acquiring a multi-channel signal by electro-encephalography (EEG) and/or magnetoencephalography (MEG) and/or for processing a multi-channel signal acquired by electroencephalography (EEG) and/or magnetoencephalography (MEG), the device comprising at least an input/output unit, a storage unit allowing to store acquired multi-channel signals, a processing unit allowing to process acquired multi-channel signals, **characterised by the fact that** the device is equipped with means adapted to perform the method for removal of artifacts from EEG/MEG multi-channel signals according to one of claims 1 to 13, the input/output unit preferably comprising a set of electrodes adapted to acquire a EEG/MEG multi-channel signal.
